# EUROPEAN PATENT SPECIFICATION

(11) **EP 2 480 682 B1**
(45) Date of publication and mention of the grant of the patent: **31.08.2016**
(21) Application number: 10754882.8
(22) Date of filing: 14.09.2010
(51) Int. Cl.: C12Q 1/18

(54) **PAPER STRIP FOR DETERMINING MINIMUM INHIBITORY CONCENTRATIONS OF ANTIBIOTICS**
PAPIERSTREIFEN ZUR BESTIMMUNG VON MINIMALEN HEMMENDEN ANTIBIOTIKAKONZENTRATIONEN
BANDELETTE DE PAPIER POUR DÉTERMINER DES CONCENTRATIONS INHIBITRICES MINIMALES D'ANTIBIOTIQUES

(30) Priority: 21.09.2009 IT AN20090064
(43) Date of publication of application: 01.08.2012
(73) Proprietor: Liofilchem S.r.l., 64026 Roseto Degli Abruzzi (TE) (IT)
(72) Inventor: BROCCO, Silvio, I-64026 Roseto Degli Abruzzi (TE) (IT)
(74) Representative: Statti, Francesco
(86) International application number: PCT/EP2010/005644
(87) International publication number: WO 2011/032683

(56) References cited:
- EP-A1- 0 157 071
- US-A- 4 778 758
- OXOID LTD.: "M.I.C. Evaluators (M.I.C.E.) Simple, Convenient Method for Accurate MIC Values", RAPIDMICROBIOLOGY NEWS, [Online] 30 May 2008 (2008-05-30), pages 1-2, XP002577887, Retrieved from the Internet: URL:http://www.rapidmicrobiology.com/news/ 603h277.php> [retrieved on 2010-04-15]
- TRISTRAM STEPHEN G: "A comparison of Etest (R), MICEvaluator (TM) strips and CLSI broth microdilution for determining beta-lactam antimicrobial susceptibility in Haemophilus influenzae", JOURNAL OF ANTIMICROBIAL CHEMOTHERAPY, vol. 62, no. 6, December 2008 (2008-12), pages 1464-1466, XP002577986, ISSN: 0305-7453
- NORIEGA LUIS ET AL: "ACQUISITION OF BILE SALT RESISTANCE PROMOTES ANTIBIOTIC SUSCEPTIBILITY CHANGES IN BIFIDOBACTERIUM", JOURNAL OF FOOD PROTECTION, INTERNATIONAL ASSOCIATION FOR FOOD PROTECTION, US, vol. 68, no. 9, 1 January 2005 (2005-01-01), pages 1916-1919, XP008068975, ISSN: 0362-028X
- JAMES VERSALOVIC ED - MCMILLAN J A ET AL: "CHAPTER 146: Diagnostic Microbiology for Pediatric Infections", 17 April 2006 (2006-04-17), OSKI'S PEDIATRICS. PRINCIPLES & PRACTICE (FOURTH EDITION), LIPPINCOTT WILLIAMS & WILKINS, PAGE(S) 1002 - 1009, XP009171247, ISBN: 0-7817-3894-6
- SMAILL F: "ANTIBIOTIC SUSCEPTIBILITY AND RESISTANCE TESTING: AN OVERVIEW", CANADIAN JOURNAL OF GASTROENTEROLOGY, MEDICARE PUBL., OAKVILLE, CA, vol. 14, no. 10, 1 November 2000 (2000-11-01), pages 871-875, XP008039641, ISSN: 0835-7900

## Description

### Field of invention

The present invention concerns the sector of chemical, clinical, bacteriological and immunological analyses and more specifically it concerns the use of a strip of impregnated paper in order to determine, in the simplest, most economical and most accurate way, the correct Minimum Inhibitory Concentration (M.I.C.) of antibiotic molecules to be administered to patients.

### Background of invention

One of the main objectives of medicine is the determination of the correct dosage of antibiotics required both to obtain the greatest effect in order to eradicate the infection and to prevent large doses of antibiotics from having adverse reactions for the patient.

Initially, this problem was resolved in medicine by means of the CLSI (Clinical and Laboratory Standard Institute) standardized microdilution method for dosing the antibiotic with progressively decreasing concentrations. The problem with this method is that it takes a very long time to determine the Minimum Inhibitory Concentration (M.I.C.), due to the need to carry out numerous manual laboratory tests in order to identify the correct dose of antibiotic to be administered to the patient.

In order to remedy this problem, a patent exists in prior art, European Patent No. EP 0 157 071, application No. 84850107.8 of 3 April 1984, entitled *"Method and device for producing varying concentration patterns of chemically or biologically active substances*", the twenty-year term of which has expired, and which claimed a new method for the determination of the Minimum Inhibitory Concentration (M.I.C.) to be administered to the patient, using a graded carrier with a scale of concentrations of one or more clinically or biologically active substances, so that the substance on this carrier is transferred onto the surface of a culture medium containing bacteria taken from the patient, making it possible to determine the Minimum Inhibitory Concentration (M.I.C.).

On expiry of patent EP 0 157 071, the English company OXOID Ltd. launched the M.I.C. Evaluator (M.I.C.E.) on the market. This product is based on the same principle as the aforementioned patent, using a polymer strip graded with a concentration scale of numerous clinically or biologically active substances.

Both in the patent EP 0 157 071 and for the M.I.C Evaluator manufactured by OXOID, the carriers are made of plastic and are difficult to remove from the wrapping which contains them due to the fact that they are very thin, making them more sensitive to the electrostatic attraction forces typical of thin plastic layers and, in order to overcome this problem, the use of specific and expensive equipment is required.

Another problem is that the plastic material of which the carrier is made is impermeable to air; as a result incorrect applications to the microbial culture medium can create air bubbles which may not be visible to the naked eye but which could invalidate the test, or render it inaccurate.

A further problem of the plastic carrier is that as soon as it comes into contact with the agar surface of the microbial culture medium, it immediately starts releasing the antibiotics with which it is impregnated, thereby making its correct repositioning on the microbial culture medium impossible.

Other earlier patents, such as patent No. US 4 778 758, describe and claim a device for the determination of microorganisms consisting of a sealed rectangular container containing a transparent non porous strip for the determination of the test (see Claim 1 page 4, column 4, third last line).

US 4 778 758 discloses and claims *"a rectangular, transparent, non porous, inert test strip "* (see column 2, lines 50-51; claims 1-2) and explains in claim 11 that said test strip is made of an *"inert polymeric material selected from the group consisting of acrylic plastic material, styrene plastic material and vinyl chloride plastic material ".*

### Disclosure of invention

The object of the present invention is to determine the Minimum Inhibitory Concentration (M.I.C.) using a paper strip printed with a known numerical sequence, corresponding to a different grading of the impregnated antibiotic, so as to simplify its extraction from the blister or the wrapping which normally protects it and avoid the creation of air bubbles between the microbial cultural medium and the paper strip itself, which could invalidate the test or render it inaccurate.

Another object of the present invention is to have a paper strip impregnated with antibiotics according to a graded scale that has the characteristic of gradually releasing the antibiotics once positioned in the microbial culture medium, so as to facilitate its repositioning on the microbial culture medium. Another object of the present invention is to reduce the cost of the method for determining the Minimum Inhibitory Concentration (M.I.C.).

These and other objects are achieved by the paper strip as defined in claim 1. Preferred embodiments thereof are described in the dependent claims. These and other objects may be in particular achieved by the invention that is the object of the present application which concerns the sector of chemical, clinical, bacteriological and immunological analyses and, more specifically that of the determination of the Minimum Inhibitory Concentration (M.I.C.) using a paper strip impregnated with a predetermined concentration gradient of the antimicrobial agent according to the known technique, with a view to simplifying the execution of the relative test due to the fact that the said strip of paper, being permeable to air, possesses the characteristic of releasing the antibiotics very slowly once positioned on the microbial culture medium, thereby facilitating its repositioning, if required, on the microbial culture medium.

### Brief description of drawings

Further features and advantages of the invention shall be better understood from the description of a preferred but not exclusive embodiment, illustrated by way of indicative but non-limiting example in the accompanying drawings in which:

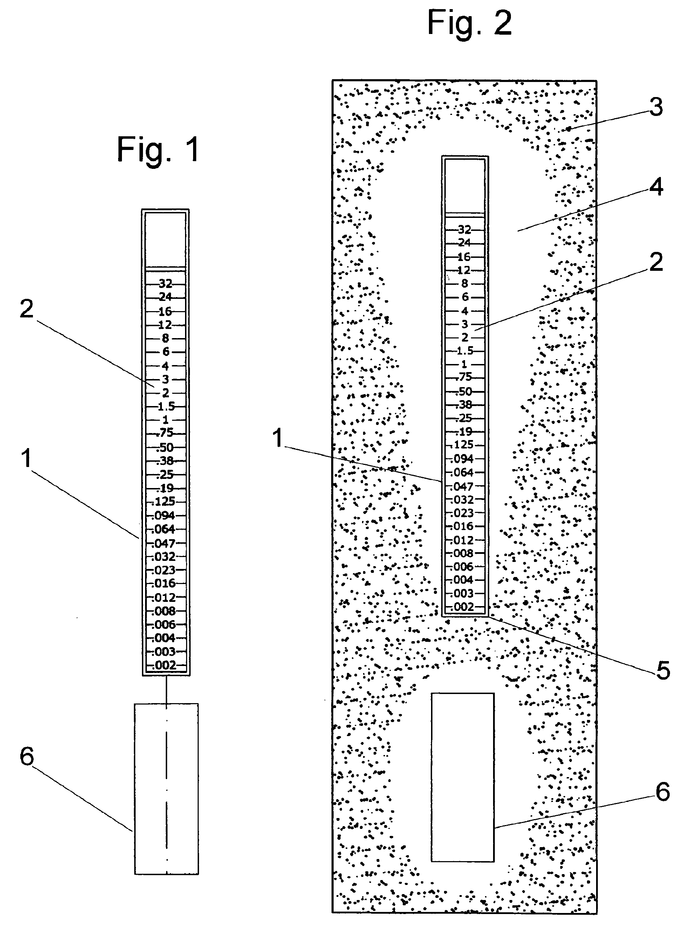

Fig. 1 represents the paper strip impregnated with the predetermined concentration gradient of the antibacterial agent, graded on a colour scale of fifteen dilution intervals expressed in µg/mL according to the known technique.
Fig. 2 represents the configuration of the zone created by the placing of the aforementioned paper carrier on the microbial culture medium, the lower part of which, at the intersection of the zone with the strip, indicates the inhibiting concentration of antibiotic required to be administered to the patient.

### Detailed description of invention

The invention that is object of the present application concerns a paper strip (1) impregnated with a predetermined concentration gradient of an antibacterial agent graded on a colour scale of fifteen dilution intervals expressed in µg/mL (2) for determining the Minimum Inhibitory Concentration (M.I.C.) at a zone (4) that intersects the strip (1), positioned on a microbial culture medium of bacteria (3) taken from the patient, at point (5) at a given value reading of the antibacterial agent, and facilitates its extraction from the relative blister or wrapping because adjacent paper strips havenot a reciprocal force of electrostatic attractionas plastic strips.

Additionally, the aforementioned paper strip (1) possesses the characteristic of being permeable to air thereby preventing air bubbles from forming at the point of contact between the microbial culture medium (3) and the paper strip (1) that could invalidate the test or render it inaccurate.

A further advantage for the user is that the paper strip (1) may possess the additional characteristic that when it first comes into contact with the microbial culture medium (3) it begins to release the antibiotics with which it is impregnated very slowly, thereby allowing its user to reposition it easily on the microbial culture medium (3) for the determination of the Minimum Inhibitory Concentration (M.I.C.).

With a view to preserving from moisture the predetermined concentrations of the antibacterial agent, shown on the colour scale of fifteen dilution intervals expressed in µg/mL (2), the paper strip (1) may be equipped with a silica gel container (6).

The materials and the dimensions of the above-described invention, illustrated in the accompanying drawings and later claimed, may be varied according to requirements. Moreover, all the details may be replaced by other technically equivalent ones without for this reason straying from the protective scope of the present invention patent application.

## Claims

1. Paper strip (1) for determining Minimum Inhibitory Concentration of antibiotics, **characterized by** the fact that,
it is made from paper being permeable to air, it prevents air bubbles from forming at the point of contact with the microbial culture medium (3) that could invalidate the test or render it inaccurate and that it has a predetermined concentration gradient of antibacterial agent graded on a scale of fifteen dilution intervals.

2. Paper strip (1) for determining Minimum Inhibitory Concentration of antibiotics according to claim 1, **characterized by** the fact that the aforementioned paper strip (1) on initial contact with the microbial culture medium (3) begins to release the antibiotics with which it is impregnated very slowly and gradually facilitating the user should the strip require repositioning on the microbial culture medium (3).

3. Paper strip (1) for determining Minimum Inhibitory Concentration of antibiotics according to any one of claims 1 or 2, **characterized by** the fact that it is equipped with a silica gel container (6) in order to protect from moisture the predetermined concentrations of the antibacterial agent shown on the colour scale of fifteen dilution intervals expressed in µg/mL (2).

4. Paper strip (1) for determining Minimum Inhibitory Concentration of antibiotics according to any one of claims 1 to 3, **characterized in that** the paper strip (1) comprises a colour scale.

5. Paper strip (1) for determining Minimum Inhibitory Concentration of antibiotics according to any one of claims 1 to 4, **characterized in that** the fifteen dilution intervals are expressed in µg/mL.

6. Paper strip (1) for determining Minimum Inhibitory Concentration of antibiotics according to any one of claims 1 to 5, **characterized in that** it is impregnated with the predetermined concentration gradient of the antibacterial agent.

## Patentansprüche

1. Papierstreifen(1) zur Bestimmung von minimalen hemmenden Antibiotikakonzentrationen, der **dadurch gekennzeichnet ist, dass**
er aus luftdurchlässigem Papier besteht, und verhindert, dass sich Luftblasen am Kontaktpunkt mit dem mikrobiellen Kulturmedium (3) bilden, welche den Test ungültig oder ungenau machen würden, und dass er einen vorgegebenen Konzentrationsgradienten an antibakteriellem Wirkstoff hat, welcher auf einer Skala von fünfzehn Verdünnungs-Abständen eingestuft ist.

2. Papierstreifen (1) zur Bestimmung von minimalen hemmenden Antibiotikakonzentrationen nach Anspruch 1, die **dadurch gekennzeichnet sind, dass** der vorgenannte Papierstreifen (1) bei Erstkontakt mit dem mikrobiellen Kulturmedium (3) beginnt, die Antibiotika freizugeben, mit denen er sehr langsam und graduell imprägniert ist und dem Benutzer erleichtern, sollte der Streifen eine Neupositionierung auf dem mikrobiellen Nährmittel(3)erfordern.

3. Papierstreifen (1) zur Bestimmung von minimalen hemmenden Antibiotikakonzentrationen nach einem der Ansprüche 1 oder 2, der **dadurch gekennzeichnet ist, dass** er mit einem Silikagel-Behälter (6) ausgestattet ist, um die vorgegebenen Konzentrationen an antibakteriellem Wirkstoff vor Feuchtigkeit zu schützen, die auf der Farbskala von fünfzehn Verdünnung-Abständen gezeigt sind und in µg/mL (2) angegeben werden.

4. Papierstreifen (1) zur Bestimmung von minimalen hemmenden Antibiotikakonzentrationen nach einem der Ansprüche 1 bis 3, der **dadurch gekennzeichnet ist, dass** der Papierstreifen (1) eine Farbskala enthält.

5. Papierstreifen (1) zur Bestimmung von minimalen hemmenden Antibiotikakonzentrationen nach einem der Ansprüche 1 bis 4, der **dadurch gekennzeichnet ist, dass** die fünfzehn Verdünnungs-Abstände in µg/mL angegeben werden.

6. Papierstreifen (1) zur Bestimmung von minimalen hemmenden Antibiotikakonzentrationen nach einem der Ansprüche 1 bis 5, der **dadurch gekennzeichnet ist, dass** er mit dem vorgegebenen Konzentrationsgradienten des antibakteriellen Wirkstoffs imprägniert ist.

## Revendications

1. Bandelette de papier (1) pour déterminer des concentrations inhibitrices minimales d'antibiotiques, **caractérisée par le fait**
**que** celle-ci est fabriquée dans un papier perméable à l'air, qu'elle empêche la formation de bulles d'air au point de contact avec le milieu de culture microbienne (3) susceptibles d'invalider l'essai ou d'en fausser les résultats et qu'elle est imprégnée d'un gradient de concentration prédéfini d'agent antibactérien disposé sur une échelle graduée de quinze intervalles de dilution.

2. Bandelette de papier (1) pour déterminer des concentrations inhibitrices minimales d'antibiotiques selon la revendication 1, **caractérisée par le fait que** la bandelette de papier (1) précitée, lors de son contact initial avec le milieu de culture microbienne (3), commence à libérer très lentement et progressivement les antibiotiques dont elle est imprégnée, en facilitant le travail de l'utilisateur dans le cas où la bandelette devrait être repositionnée sur le milieu de culture microbienne (3).

3. Bandelette de papier (1) pour déterminer des concentrations inhibitrices minimales d'antibiotiques selon l'une quelconque des revendications 1 ou 2, **caractérisée par le fait que** celle-ci est dotée d'un conteneur de silicagel (6) pour protéger de l'humidité les concentrations prédéfinies de l'agent antibactérien, qui apparaissent sur l'échelle colorimétrique comportant quinze intervalles de dilution exprimés en µg/mL (2).

4. Bandelette de papier (1) pour déterminer des concentrations inhibitrices minimales d'antibiotiques selon l'une quelconque des revendications 1 à 3, **caractérisée par le fait que** la bandelette de papier (1) comprend une échelle colorimétrique.

5. Bandelette de papier (1) pour déterminer des concentrations inhibitrices minimales d'antibiotiques selon l'une quelconque des revendications 1 à 4, **caractérisée par le fait que** les quinze intervalles de dilution sont exprimés en µg/mL.

6. Bandelette de papier (1) pour déterminer des concentrations inhibitrices minimales d'antibiotiques selon l'une quelconque des revendications 1 à 5, **caractérisée par le fait que** celle-ci est imprégnée d'un gradient de concentration prédéfini d'agent antibactérien.
